# EUROPEAN PATENT APPLICATION

(11) **EP 1 941 928 A1**
(43) Date of publication of application: **09.07.2008**
(21) Application number: 06811031.1
(22) Date of filing: 02.10.2006
(51) Int. Cl.: A61N 1/30, A61N 1/04

(54) **ELECTRODE STRUCTURE FOR IONTOPHORESIS USED TO ADMINISTER DRUG ENCLOSED IN NANOPARTICLE AND IONTOPHORESIS DEVICE MAKING USE OF THE SAME**

(30) Priority: 30.09.2005 JP 2005288148
(71) Applicant: TTI ELLEBEAU, INC., Tokyo 140-0002 (JP)
(72) Inventor: AKIYAMA, Hidero c/o TTI ellebeau, Inc., Tokyo 150-0022 (JP); NAKAYAMA, Mizuo c/o TTI ellebeau, Inc., Tokyo 150-0022 (JP); MATSUMURA, Takehiko c/o TTI ellebeau, Inc., Tokyo150-0022 (JP); MATSUMURA, Akihiko c/o TTI ellebeau, Inc., Tokyo150-0022 (JP)
(74) Representative: Liesegang, Eva
(86) International application number: PCT/JP2006/319683
(87) International publication number: WO 2007/037474

(57) **Abstract**

The present invention relates to an electrode assembly for iontophoresis which enables iontophoresis administration of a drug that does not dissociate into ions, or that is insoluble or poorly-soluble in water, and which makes it possible to provide the drug with the functionality in drug delivery. More specifically, the present invention relates to the electrode assembly for iontophoresis which comprises a drug enclosed in an ionic nanoparticle.

## Description

### CROSS-REFERENCE TO RELATED APPLICATIONS

This application claims the benefit of priority to Japanese Patent Application No. 2005-288148 (filed on September 30, 2005), the entire disclosure of which is incorporated herein by reference.

### BACKGROUND OF THE INVENTION

### Field of Invention

The present invention generally relates to the field of iontophoresis, and in particular, to an iontophoresis electrode assembly for delivering a drug enclosed in an ionic nanoparticle to a subject, and to an iontophoresis device that uses the electrode assembly.

### Background Art

Iontophoresis refers to a method of delivering an ionic drug placed on the surface of a biological interface, such as skin or mucosa, of a subject and into the subject's body by use of an electromotive force sufficient to drive the ionic drug. Refer to JP 63-35266 A for one example of iontophoresis.

Positively charged ions may be driven (transported) into a biological interface on an anode (positive electrode) side of an iontophoresis device, for example. Negatively charged ions may be driven into a biological interface on a cathode (negative electrode) side.

Several iontophoresis devices as described above have been proposed (see, for example, JP 63-35266 A, JP 04-297277 A, JP 2000-229128 A, JP 2000-229129 A, JP 2000-237327 A, JP 2000-237328 A, WO 03/037425 A1, JP 2004-518707 A, JP 2004-231575 A, and JP 2003-501379 A).

It may be difficult, however, to apply iontophoresis to delivery of a drug that does not dissociate into ions, that is insoluble in water or fat-soluble, or that has a high molecular weight. The number of potential drugs deliverable by conventional iontophoresis may thus be limited. Further, it has been desired to provide a drug administered by iontophoresis with a functionality to be delivered to specific areas in a subject.

Accordingly, it is an important problem to enable iontophoresis application of a drug that does not dissociate into ions, or that is insoluble or poorly-soluble in water. Also, it is a problem to provide a drug with the functionality in drug delivery.

### SUMMARY OF THE INVENTION

The present invention has been completed in light of the problems of the prior art. Accordingly, an object of the present invention is to provide an electrode assembly for iontophoresis and an iontophoresis device using the same, which make it possible to administer by iontophoresis a drug that does not dissociate into ions, or that is insoluble or poorly-soluble in water, and which make it possible to provide the drug, including ionic drug, with the functionality in drug delivery.

To solve the above mentioned problems, the electrode assembly for iontophoresis of the present invention comprises a drug enclosed in an ionic nanoparticle.

In a preferred aspect, the electrode assembly of the present invention at least comprises an electrode coupled to an electric power source device having the same polarity as that of the ionic nanoparticle in the electrode assembly, an electrolyte solution reservoir impregnated with an electrolyte solution, the electrolyte solution reservoir being placed adjacent to the electrode, an ion exchange membrane selective to ions with a polarity opposite to that of the ionic nanoparticle, the ion exchange membrane being placed adjacent to the electrolyte solution reservoir, a drug solution reservoir impregnated with the ionic nanoparticle, the drug solution reservoir being placed adjacent to the ion exchange membrane, and an ion exchange membrane selective to ions with the same polarity as that of the ionic nanoparticle, the ion exchange membrane being placed adjacent to the drug solution reservoir.

In a preferred aspect, the electrode assembly of the present invention is the one wherein the drug solution reservoir includes pores capable of holding and passing the ionic nanoparticle.

In a preferred aspect, the electrode assembly of the present invention is the one wherein the ion exchange membrane which is selectively permeable to ions having the same polarity as that of the ionic nanoparticle includes pores capable of passing the ionic nanoparticle, and the ion exchange membrane which is selectively permeable to ions having a polarity opposite to that of the ionic nanoparticle does not include pores capable of passing the ionic nanoparticle.

In a preferred aspect, the electrode assembly of the present invention is the one wherein the ionic nanoparticle is a cationic nanoparticle.

In another preferred aspect, the electrode assembly of the present invention is the one wherein the ionic nanoparticle is an anionic nanoparticle.

In a preferred aspect, the electrode assembly of the present invention is the one wherein the drug enclosed in the ionic nanoparticle is selected from cancer therapeutic agents, nucleic acids such as genes, and peptides.

In a preferred aspect, the iontophoresis device of the present invention comprises an electric power source device, a drug administration means comprising two or more electrode assemblies, at least one of the electrode assemblies comprising a drug enclosed in an ionic nanoparticle, and a current control means for controlling current flow to each of the electrode assemblies, the current flowing from the current control means causing the electrode assembly to release the ionic nanoparticle so as to be administered transdermally to a subject.

In a preferred aspect of the present invention, the iontophoresis device is the one wherein the drug administration means is configured integrally.

According to the present invention, the electrode assembly comprises a drug enclosed in an ionic nanoparticle, and thus it become possible to administer by iontophoresis a drug that does not dissociate into ions or that is insoluble or poorly-soluble in water , and to provide the drug with the functionality in drug delivery.

### BRIEF DESCRIPTION OF THE DRAWINGS

Figure 1 shows a frame format of an electrode assembly for iontophoresis.
Figure 2 shows a frame format of an iontophoresis device that includes an electrode assembly for iontophoresis.

### DETAILED DESCRIPTION OF THE INVENTION

As mentioned above, the electrode assembly for iontophoresis of the present invention comprises a drug enclosed in an ionic nanoparticle.

As used herein, the term "nanoparticle" refers to a hollow particle having a diameter of 4 nm to 400 nm and capable of enclosing a substance therein.

Ionic nanoparticles have positively or negatively charged functional groups on a surface film thereof. This charged nature allows ionic nanoparticles to be administered via iontophoresis. For example, a cationic nanoparticle may have a functional group such as -NH³⁺. The functional group can reinforce an interaction with a negatively charged biological cell. An anionic nanoparticle, by way of comparison, may have a functional group such as a carboxyl group, so interactions with negatively charged biological cell can be suppressed.

Non-limiting examples of materials that may be used to form ionic nanoparticles include PLGA (polylactic acid-glycolic acid) and PLA (polylactic acid). Use of such a material may delay the release of a drug as the molecular weight of the ionic nanoparticle becomes larger. In addition, the period of time during which a drug is effective may be lengthened due to sustained release properties found when using such materials. This may help reduce any burden placed on a subject. Also, ionic nanoparticles may be coated with lecithin, for example. The coated ionic nanoparticles may accumulate on a portion of a biological interface that is inflamed or the like, thereby enabling targeted delivery. In addition, such ionic nanoparticles may be susceptible to deformation, similar to red blood cells, through energization, thus allowing the ionic nanoparticles to pass through and be absorbed by an ion exchange membrane or a biological interface.

Ionic nanoparticles enclosing a drug therein may be formed by using any of a variety of methods, some of which are outlined below. The following method may be used when a water soluble drug is to be enclosed in ionic nanoparticles. The drug may be dissolved in acetone along with PLGA (or PLA), and zinc acetate may be added to the solution. The resultant may then be added dropwise to water, and lecithin may then be added, thus forming nanospheres. A specific example follows.

1 mg of triptorelin may be dissolved into 100 µL of an aqueous solution (pH 7, W1). The solution may then be emulsified with an organic solvent (ethyl acetate, dichloromethane) into which PLGA has been dissolved, and the resultant may be exposed to ultrasonic irradiation. Next, 2 mL of an aqueous solution containing 1% PVA (W2) may be added to the resultant, and the whole may be exposed to ultrasonic irradiation to prepare a W1/0/W2 emulsion. The emulsion can then be diluted with 15 mL of a 0.3% aqueous solution of PVA, and the organic solvent evaporated under reduced pressure. The resultant nanoparticles may be separated by means of ultracentrifugation (13,000 x g, 30 minutes). A resulting supernatant may be removed and washed with purified water.

Fine particles prepared by using PLGA50/50 having a large number of free carboxyl groups, each having negative charge. In addition, when prepared fine particles and cetyl butyl ammonium bromide (CTAB) are mixed and stirred overnight, CTAB adsorbs onto the surface of each particle, so the charge of each fine particle becomes positive.

The following method (solvent diffuson method) may be used when a fat-soluble drug is to be enclosed in ionic nanoparticles. PLGA (or PLA) may be dissolved in acetone along with the drug. The acetone solution may be added dropwise to an aqueous solution that includes polyvinyl alcohol, Pluronic F68, or Tween 20 while being stirred. A fine particle emulsion can thus be obtained. The emulsion may be separated by using ultracentrifugation, and a resulting supernatant may be removed and washed with purified water. Alternatively, the following method (a solvent evaporation method) may also be adopted. PLGA (or PLA) may be dissolved in 1 mL of dichloromethane along with the drug. An aqueous solution of added egg yolk lecithin and the dichloromethane solution may be mixed and stirred, and the resultant exposed to ultrasonic irradiation. The resultant may then be stirred at room temperature for 2 hours. Resultant fine particles may then be separated by using ultracentrifugation. A resultant supernatant may be removed and washed with purified water.

Examples of drugs that may be enclosed in ionic nanoparticles include those stated below. In the examples, narrowly-defined ionic drugs, i.e., the drugs itself which can dissociate into ions, can be directly administered from an electrode assembly comprising the same without being enclosed in ionic nanoparticles.

The examples of drugs include various cancer therapeutic agents, therapeutic genes, and peptides. Such drugs may be targeted to specific sites of a subject when enclosed in an ionic nanoparticle and delivered by iontophoresis. The drugs may also incorporate sustained release properties. Furthermore, the drug may be administered by using iontophoresis even if the drug itself does not readily dissociate into ions, and even if the drug is substantially insoluble in water. When applied to an drug having a large molecular weight, a valence greater than that of the original ionic charge can be provided for the entirety of the drug by, for example, adjusting the number of functional groups on the nanoparticles, thus increasing mobility and facilitating increased transport.

Examples of cationic drugs that may be enclosed in ionic nanoparticles include: local anesthetics (such as procaine hydrochloride and lidocaine hydrochloride); gastrointestinal disease therapeutics (such as carnitine chloride); skeletal muscle relaxants (such as vancuronium bromide); and antibiotics (such as tetracycline based preparations, kanamycin based preparations, and gentamicin based preparations).

Examples of anionic drugs that may be enclosed in ionic nanoparticles include: vitamins (such as riboflavin phosphate, nicotinic acid, ascorbic acid, and folic acid); adrenal cortex hormones (such as a hydrocortisone based water soluble preparations, and dexamethasone based and prednisolone based water soluble preparations such as prednisolone sodium phosphate and dexamethasone sodium phosphate); and antibacterial agents (such as a quinolone based preparations).

Examples of vaccines that may be enclosed in ionic nanoparticles include BCG vaccine, hepatitis A vaccine, melanoma vaccine, measles vaccine, poliomyelitis vaccine, and influenza vaccines.

Examples of adjuvants that may be enclosed in ionic nanoparticles include MPL (Monophosphoryl lipid A), DMPC (dimyristoylphosphatidylcholine), QS-21, DDA (Dimethyl dioctadecyl ammonium chloride), and RC-529.

Furthermore, combinations of a vaccine and an adjuvant may also be enclosed, such as: a combination of a positively ionized vaccine and RC-529; a combination of a negatively ionized vaccine and DDA; a combination of a BCG vaccine and MPL; a combination of a hepatitis A vaccine and DMPC; and a combination of a melanoma vaccine and QS-21.

Other combinations of drugs may also be used, such as: a combination of a hypotensive drug and a hypotensive diuretic agent, for example lisinopril and hydrochlorothiazide, methyldopa and hydrochlorothiazide, clonidine hydrochloride and chlorthalidone, and benazepril hydrochloride and hydrochlorothiazide; a combination containing an antidiabetic agent, such as insulin and metformin hydrochloride; and other combinations such as ozagrel hydrochloride and ozagrel sodium, and codeine hydrochloride and promethazine hydrochloride.

Various types of the ionic drugs (ionic nanoparticles and narrowly-defined ionic drugs) can be used in combination depending on diseases or conditions in a subject. These various ionic drugs may exist separately in different electrode assemblies or may be combined together in one electrode assembly.

The amount of each ionic drug present may be set to effect a desired blood concentration upon application to a subject over a certain period of time. The amount may be set by one skilled in the art in accordance with, for example, the size and/or thickness of a drug solution reservoir, the size of an drug release surface area, the size of an electric potential applied, administration time, or the like.

An inactive electrode made of a conductive material such as carbon or platinum can be preferably used as the electrode of the electrode assembly.

The electrolyte solution reservoir can be composed of a thin film that can be impregnated with an electrolyte solution. The thin film can be made of the same material as that used for a drug solution reservoir described later, which is impregnated with an ionic drug. A desired one can be appropriately used as the electrolyte solution depending upon the conditions such as a drug to be applied; however, a solution that damages the skin of a subject owing to the electrode reaction should be avoided. An organic acid or a salt thereof present in a metabolic cycle of the subject is preferable for the electrolyte solution in the present invention in terms of harmlessness. Typical examples include lactic acid and fumaric acid. Specifically, an aqueous solution of 1M of lactic acid and 1M of sodium fumarate (1 : 1) may be used. Such electrolyte solution is preferable because it has high solubility with respect to water, pass current well, and show small changes in pH at a constant current.

The drug solution reservoir may comprise a thin film which can be impregnated with an ionic drug or the like. It is important that the film have a sufficient ability of being impregnated with an ionic drug, and a sufficient ability (ion transferability, ion conductivity) of moving the ionic drug to the skin side under predetermined electric field conditions.
Examples of a material having both the satisfactory impregnation characteristics and the satisfactory ion conductivity include hydrogel forms of acrylic resins (acrylhydrogel film), segmented polyurethane based gel matrix films, and ion conductive porous sheets for forming a gel matrix-like solid electrolyte (refer to a porous polymer disclosed in Japanese Patent Laid-Open Publication 273452/1936 A using, as a base, an acrylonitrile copolymer containing 50 mol% or more, preferably 70 to 98 mol% or more of acrylonitrile and having a porosity of 20 to 80%). In case that the drug solution reservoir is impregnated with the solution, impregnation rate thereof (defined as (W-D)/D, where D indicates dry weight and W indicates weight after impregnation) is preferably 30 to 40%.

In addition, the drug solution reservoir may have pores capable of holding ionic nanoparticles, and allowing ionic nanoparticles to pass therethrough. The drug solution reservoir having such porous structure can easily hold ionic nanoparticles by being immersed in a liquid containing the ionic nanoparticles, or aspirating a liquid containing the ionic nanoparticles therethrough.

Cation exchange membrane and Anion exchange membrane is preferably used in combination for the electrode assembly. Examples of cation exchange membrane include NEOSEPTAs (CM-1, CM-2, CMX, CMS, CMB, and CLE04-2) manufactured by Tokuyama Co., Ltd. Examples of anion exchange membrane include NEOSEPTAs (AM-1, AM-3, AMX, AHA, ACH, ACS, ALE04-2, and AIP-21) manufactured by Tokuyama Co., Ltd. Cation exchange membranes may comprise a porous film having cavities, a portion or the entirety of which are filled with an cation exchange resin. Anion exchange membranes may comprise a porous film having cavities, a portion or the entirety of which are filled with an anion exchange resin.

Ion exchange membranes may have pores through which ionic nanoparticles can pass. In particular, an ion exchange membrane having the same polarity as that of the ionic nanoparticles used may have pores through which the ionic nanoparticles can pass, while an ion exchange membrane having a polarity opposite that of the ionic nanoparticles may comprise a non-porous membrane, allowing the ionic nanoparticles to be effectively supplied toward a biological interface.

The ion exchange resins may be fluorine based and include a perfluorocarbon skeleton having an ion exchange group, or may be hydrocarbon based and include a nonfluorinated resin as a skeleton. Hydrocarbon based ion exchange resins are preferably used because these resins are easier to manufacture. The filling rate of the porous film by the ion exchange resin varies depending on the porosity of the porous film, and the rate may be 5 to 95 % by mass, preferably 10 to 90 % by mass, more preferably 20 to 60 % by mass.

Ion exchange groups in the ion exchange resins are not limited as far as these functional groups have negative or positive charge when in aqueous solution. The functional groups may also be present in the form of a free acid or a salt. Examples of cation exchange groups include sulfonic groups, carboxylic acid groups, and phosphonic acid groups. Examples of counter cations for the cation exchange group include: alkali cations such as sodium ions and potassium ions, and ammonium ions. Examples of anion exchange groups include primary amino groups, secondary amino groups, tertiary amino groups, quaternary ammonium groups, pyridyl groups, imidazole groups, quaternary pyridium groups, and quaternary imidazolium groups. Examples of counter cations for the anion exchange group include: halogen ions such as chlorine ions, and hydroxy ions.

In addition, any film or sheet which has pores passing therethrough can be used as the porous film without specific limitations. However, to satisfy both of high strength and flexibility, it may be advantageous that the porous film is made of a thermoplastic resin. Examples of the thermoplastic resins include: polyolefin resins such as homopolymers or copolymers of α-olefins, such as ethylene, propylene, 1-butene, 1-pentene, 1-hexene, 3-methyl-1-butene, 4-methyl-1pentene, and 5-methyl-1-heptene; vinyl chloride based resins such as polyvinyl chloride, vinyl chloride-vinyl acetate copolymers, vinyl chloride-vinylidene chloride copolymers, and vinyl chloride-olefin copolymers; fluorine based resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 66; and polyimide resins. Further, in view of mechanical strength, flexibility, chemical stability, and chemical resistance, the thermoplastic resins is preferably polyolefin resins, more preferably polyethylene or polypropylene, still more preferably polyethylene.

Further, the mean pore size of the porous films may be preferably from 0.005 to 5.0 µm, more preferably from 0.01 to 2.0 µm, still more preferably from 0.02 to 0.2 µm. Mean pore size as used herein indicates mean flow pore size measured in conformance with the bubble point method (JIS-K3832-1990).

Further, the porosity of the porous film may be preferably from 20 to 95%, more preferably from 30 to 90%, still more preferably from 30 to 70%. In consideration of the thickness of an ion exchange membrane finally-formed, the thickness of the porous film may be preferably from 5 to 140 µm, more preferably 10 to 130 µm, still more preferably 15 to 55 µm. An anion exchange membrane or a cation exchange membrane formed by using a porous film generally has the same thickness as that of the porous film, but may also have a thickness up to about 20 µm larger than that of the porous film.

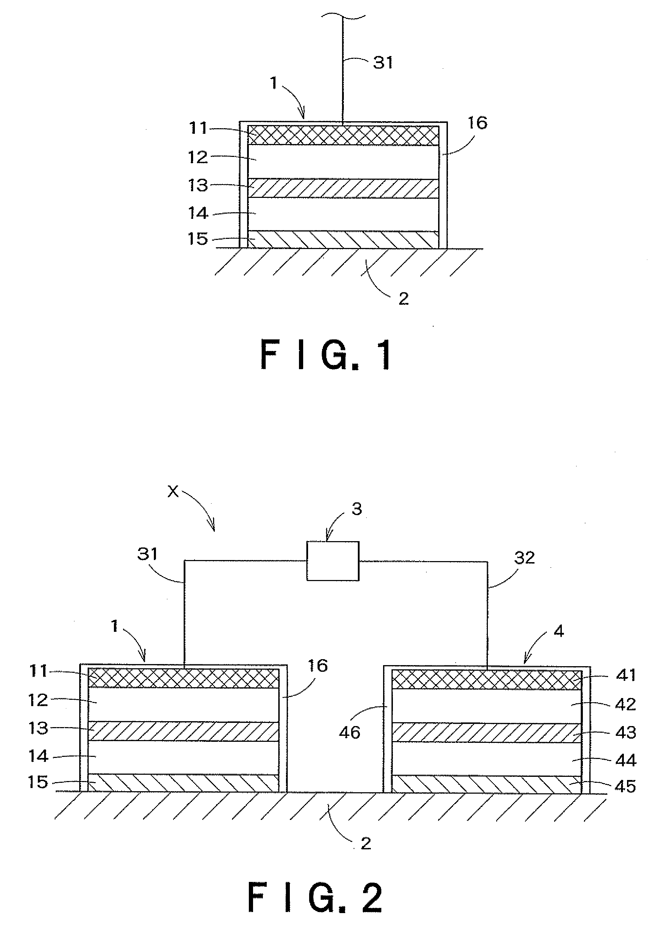

Figure 1 is a schematic view showing an electrode assembly 1 for iontophoresis arranged on a skin 2. The electrode assembly 1 may be used as an active electrode assembly for transdermally administering an ionic drug. The electrode assembly 1 may comprise: an electrode 11 coupled via the electric cable 31 to an electric power source device having the same polarity as that of ionic drug, an electrolyte solution reservoir 12 impregnated with an electrolyte solution, the electrolyte solution reservoir 12 being placed adjacent to the electrode 11, an ion exchange membrane 13 which is selectively permeable to ions with a polarity opposite to that of the ionic drug, the ion exchange membrane 13 being placed adjacent to the electrolyte solution reservoir 12, a drug solution reservoir 14 impregnated with the ionic nanoparticles, the drug solution reservoir 14 being placed adjacent to the ion exchange membrane 13, and an ion exchange membrane 15 which is selectively permeable ions with the same polarity as that of the ionic drug, the ion exchange membrane 15 being placed adjacent to the drug solution reservoir 14. A cover or container 16 is used to house the electrode assembly 1.

Figure 2 is a schematic view showing an iontophoresis device X disposed on the skin 2. The iontophoresis device X comprising: the electrode assembly (active electrode assembly) 1 for iontophoresis; an electric power source device 3; and inactive electrode assembly 4 as a counter electrode assembly.

The electrode assembly 1 for iontophoresis is coupled via the electric cable 31 to a side of the electric power source device 3 having the same polarity as that of the drug. The inactive electrode assembly 4 may comprise: an electrode 41 coupled via an electric cable 32 to a side of the electric power source 3 having a polarity opposite to the charge of an ionic drug; an electrolyte solution reservoir 42 impregnated with an electrolyte solution, the electrolyte solution reservoir 42 being placed adjacent to the electrode 41; an ion exchange membrane 43 which is selectively permeable to ions with the same polarity as that of the ionic drug, the ion exchange membrane 43 being placed adjacent to the electrolyte solution reservoir 42; an electrolyte solution reservoir 44 impregnated with an electrolyte solution, the electrolyte solution reservoir 44 being placed adjacent to the ion exchange membrane 43; and an ion exchange membrane 45 which is selectively permeable to ions having a polarity opposite that of the ionic drug, the ion exchange membrane 45 being placed adjacent to the electrolyte solution reservoir 44. The entirety of the inactive electrode assembly 4 may be housed in a cover or container 46. The inactive electrode assembly 4 is one preferable embodiment, and may also take on other configurations. For example, the electrolyte solution reservoir 42 and the ion exchange membrane 43 which is selectively permeable to ions with the same polarity as that of the ionic drug may be omitted.

When an electrode assembly holding an ionic drug is energized by the electric power source device 3, the ionic drug may move to a side opposite the electrode due to electrophoresis caused by an electric field, and thus be transdermally administered to a subject via the ion exchange membrane 15. The ion exchange membrane 13 disposed on the electrode side selectively passes ions having a polarity opposite that of the ionic drug, thus substantially preventing movement of the ionic drug toward the electrode. Having the above configuration, the electrode assembly of the present invention enables to prevent skin from being damaged by electrochemical reaction and thus safe administration of ionic drugs is achieved. Furthermore, the following conditions are preferably adopted as the operating conditions in the iontophoresis device. Constant current condition, specifically, 0.1 to 0.5 mA/cm², preferably, 0.1 to 0.3 mA/cm². Safe voltage condition that realizes the above constant current, specifically, 50 V or less, preferably 30 V or less.

Further, in the present invention, a configuration that includes a plurality of active (or inactive) electrode assemblies may also be employed. A plurality of different types of ionic drugs may thus be held by the active electrode assemblies, and the ionic drugs comprise at least one ionic nanoparticle which encloses a drug. Further, in case of administration of two or more ionic drugs which have different charges each other, active electrode assemblies and inactive electrode assemblies may be placed not only on an anode side but also on a cathode side of an iontophoresis device.

Further, a plurality of electrode assemblies may be configured as drug administering means and assembled integrally in one package for convenience of handling, for example. No particular limitations are placed on packaging materials in this case, provided that the packaging material does not substantially affect the administration of an ionic drug. One example of a packaging material is polyolefin for medical device. Furthermore, current control means may be provided in order to administer a predetermined amount of an drug within a predetermined time period. The drug administering means, the current control means, and an electric power source device may be configured integrally. For example, an iontophoresis device may be downsized in case that a button battery is used as the electric power source device, and an IC chip is used as the current control means.

In addition, the total number of electrode assemblies, as well as the combination of active electrode assemblies and inactive electrode assemblies, can be altered without losing enablement requirement of the present invention. Those skilled in the art may arrange such configurations based on the above mentioned specific example.

WO 03/037425 A1, the contents of which are hereby incorporated by reference in their entirety, by the applicant of the present disclosure describes specific elements in more detail.

## Claims

1. An electrode assembly for iontophoresis comprising an drug enclosed in an ionic nanoparticle.

2. The electrode assembly according to claim 1, at least comprising:
an electrode coupled to an electric power source device having the same polarity as that of ionic nanoparticle in the electrode assembly;
an electrolyte solution reservoir impregnated with an electrolyte solution, the electrolyte solution reservoir being placed adjacent to the electrode;
an ion exchange membrane which is selectively permeable to ions with a polarity opposite to that of the ionic nanoparticle, the ion exchange membrane being placed adjacent to the electrolyte solution reservoir;
a drug solution reservoir impregnated with the ionic nanoparticle, the drug solution reservoir being placed adjacent to the ion exchange membrane; and
an ion exchange membrane which is selectively permeable to ions with the same polarity as that of the ionic nanoparticle, the ion exchange membrane being placed adjacent to the drug solution reservoir.

3. The electrode assembly according to claim 2, wherein the drug solution reservoir includes pores capable of holding and passing the ionic nanoparticle.

4. The electrode assembly according to claim 2, wherein the ion exchange membrane which is selectively permeable to ions having the same polarity as that of the ionic nanoparticle includes pores capable of passing the ionic nanoparticle, and wherein the ion exchange membrane which is selectively permeable to ions having a polarity opposite to that of the ionic nanoparticle does not include pores capable of passing the ionic nanoparticle.

5. The electrode assembly according to claim 1, wherein the ionic nanoparticle is a cationic nanoparticle.

6. The electrode assembly according to claim 1., wherein the ionic nanoparticle is an anionic nanoparticle.

7. The electrode assembly according to claim 1, wherein the drug enclosed in the ionic nanoparticle is selected from cancer therapeutic agents, nucleic acids such as genes, and peptides.

8. An iontophoresis device comprising:
an electric power source device;
a drug administration means comprising two or more electrode assemblies, at least one of the electrode assemblies being the electrode assembly according to claim 1, the drug administration means being coupled to the electric power source device; and
a current control means for controlling a current flowing to each of the electrode assemblies, the current flowing from the current control means causing the electrode assembly to release the ionic nanoparticle so as to be administered transdermally to a subject.

9. The iontophoresis device according to claim 8, wherein the drug administration means is configured integrally.
